# EUROPÄISCHE PATENTSCHRIFT

(11) **EP 2 040 857 B1**
(45) Veröffentlichungstag und Bekanntmachung des Hinweises auf die Patenterteilung: **02.07.2014**
(21) Anmeldenummer: 07786052.6
(22) Anmeldetag: 13.07.2007
(51) Int. Cl.: B05C 17/005, A61B 17/00

(54) **FLÜSSIGKEITSSPENDER IN DER MEDIZIN IN FORM EINER HANDHABE**
LIQUID DISPENSER FOR MEDICAL PURPOSES IN THE FORM OF AN APPLICATOR
DISTRIBUTEUR DE LIQUIDE UTILISÉ EN MÉDECINE ET SE PRÉSENTANT SOUS LA FORME D'UN MANCHE

(30) Priorität: 18.07.2006 DE 102006034272
(43) Veröffentlichungstag der Anmeldung: 01.04.2009
(73) Patentinhaber: Aesculap AG, 78532 Tuttlingen (DE)
(72) Erfinder: ODERMATT, Erich, 8200 Schaffhausen (CH); SIEDLE, Gabriel, 79117 Freiburg (DE)
(74) Vertreter: Patentanwälte Ruff, Wilhelm, Beier, Dauster & Partner
(86) Internationale Anmeldenummer: PCT/EP2007/006225
(87) Internationale Veröffentlichungsnummer: WO 2008/009386

(56) Entgegenhaltungen:
- EP-A- 1 026 092
- EP-A- 1 445 032
- WO-A-01/51360
- US-A1- 2006 049 203

## Beschreibung

Die Erfindung betrifft einen Flüssigkeitsspender in der Medizin in Form einer Handhabe (Applikator) sowie seine Verwendung in der Medizin.

Der modernen Medizin steht heutzutage eine Vielzahl von unterschiedlichen Behandlungsmöglichkeiten für die Wundversorgung, insbesondere für den Wundverschluß, zur Verfügung. Routinemäßig kommen beispielsweise Nahtmaterialien und sogenannte Wundtacker zum Einsatz. Hiermit können große und insbesondere tiefe Wunden erfolgreich behandelt werden. Allerdings verursacht diese Form der Wundversorgung eine gewisse Traumatisierung des Wundmilieus. Nachteilig ist weiterhin die Entstehung von Narbengewebe, welches gerade bei oberflächlich behandelten Wunden zu kosmetisch unbefriedigenden Ergebnissen führen kann.

Daher werden alternativ zu den oben beschriebenen Wundverschlussmaterialien in zunehmendem Maße auch flüssige Klebstoffzusammensetzungen in der Wundbehandlung eingesetzt. Derartige Klebstoffzusammensetzungen basieren insbesondere auf Cyanoacrylat-Monomeren. Beispielsweise wird eine derartige Zusammensetzung unter der Bezeichnung Histoacryl^{®} von der B. Braun Melsungen AG kommerziell vertrieben.

Bei dem Cyanoacrylat-Monomeren handelt es sich um in Anwesenheit von Körperflüssigkeiten rasch aushärtende Monomere. Hierauf beruhende Klebstoffzusammensetzungen müssen daher in einer Form gelagert bzw. aufbewahrt werden, welche einer vorzeitigen Aushärtung der Klebstoffzusammensetzung vorbeugt. Gleichzeitig müssen die Klebstoffzusammensetzungen in einem sterilen Zustand auf die Wunde appliziert werden, um mögliche Wundinfektionen auszuschließen. Entsprechende Applikatorsysteme zur Verabreichung der Klebstoffzusammensetzungen müssen diesen Anforderungen gerecht werden. Zudem sollten derartige Applikatorsysteme mit einem wirtschaftlich möglichst vertretbaren Aufwand herstellbar sein.

Häufig werden Klebstoffzusammensetzungen bis zu ihrer Applikation in Glasampullen aufbewahrt bzw. gelagert. In den Glasampullen sind die Klebstoffzusammensetzungen zwar vor Feuchtigkeit und Luftsauerstoff geschützt. Allerdings haben sich Glasampullen weitgehend als umständlich bezüglich ihrer Handhabung in der medizinischen Versorgungspraxis erwiesen. Die Ampullen müssen zunächst zerbrochen werden, um die Klebstoffzusammensetzungen freizusetzen. Dies erschwert zum einen eine kontrollierte Auftragung der Zusammennetzungen, zum anderen besteht ein gewisses Verletzungsrisiko für den Chirurgen durch den Glasbruch.

Aus der EP 0 832 137 B1 ist ein Applikator für eine Klebstoffzusammensetzung aus einer inneren Glasampulle, die eine Klebstoffzusammensetzung enthält, und einer äußeren Kunststoffampulle bekannt. Durch Ausübung eines äußeren Drucks auf die Kunststoffampulle bricht die innere Glasampulle auf, wodurch die Klebstoffzusammensetzung aus der Glasampulle austritt und über eine Ausgabeöffnung auf eine Wunde aufgetragen werden kann. Damit keine Glassplitter in die Wunde gelangen können, ist die Ausgabeöffnung feinporig. Nachteilig hierbei ist jedoch, dass die Klebstoffzusammensetzung aufgrund der Porosität und Größe der Ausgabeöffnung auch an der Peripherie der Ausgabeöffnung austreten kann. Auf diese Weise ist eine kontrollierte Freisetzung der Klebstoffzusammensetzung aus dem Applikator erschwert. Eine Weiterentwicklung eines derartigen Applikatorsystems ist aus der WO 01/51218 A1 bekannt, wonach die Ausgabeöffnung mit Furchen oder Kanälen, die insbesondere porös sind, versehen sein kann. Sie sollen eine fokussierte Applikation der Klebstoffzusammensetzungen ermöglichen. Je nach Ausgestaltung der Ausgabeöffnung können derartige Applikatorsysteme nur mit einem verhältnismäßig hohen technischen Aufwand hergestellt werden.

Aus der WO 01/51360 A2 geht ein Flüssigkeitsspender mit einem äußeren Behältnis, das eine Auftragungshilfe aus einem nicht porösen Material aufweist, und einem inneren dampfdichten und geschlossenen Behältnis mit einer darin befindlichen aushärtbaren Flüssigkeit hervor.

Gegenstand der EP 1 445 032 A2 ist ein Flüssigkeitsspender mit einem äußeren Behältnis und einem inneren Behältnis in Form einer zerbrechlichen Ampulle.

Aufgabe der vorliegenden Erfindung ist es daher, ein wirtschaftlich interessantes Applikatorsystem für Klebstoffzusammensetzungen bereitzustellen, welches einerseits den ständig wachsenden Anforderungen an die Sterilität und die Lagerstabilität von Klebstoffzusammensetzungen gerecht wird und andererseits eine möglichst einfache und risikoarme Handhabung in der alltäglichen Versorgungsmedizin ermöglicht.

Diese Aufgabe wird gelöst durch einen Flüssigkeitsspender in der Medizin in Form einer Handhabe (Applikator) mit den Merkmalen von Anspruch 1.

Durch die Erfindung wird ein Applikatorsystem bereitgestellt, welches insbesondere eine kontrollierte und fokussierte Auftragung der aushärtbaren Flüssigkeit auf eine zu versorgende Wunde ermöglicht, ohne dass eine vorzeitige Aushärtung der Flüssigkeit stattfindet und/oder ohne dass das Wundmilieu in unerwünschter Weise mit anderen Komponenten des Applikatorsystems in Berührung kommt.

Die Austragsöffnung des erfindungsgemäßen Flüssigkeitsspenders kann in unterschiedlichen Formen ausgebildet sein. Beispielsweise kann es sich bei der Austragsöffnung um eine Düse, insbesondere um eine Schlitzdüse, handeln. Die Auftragungshilfe ist zweckmäßigerweise zylindrisch oder konisch ausgebildet. Vorzugsweise ist die Auftragungshilfe als Auftragungsspitze ausgebildet, insbesondere nach Art einer Kanüle oder Pipettenspitze, vorzugsweise nach Art einer Kanüle.

Die Austragsöffnung kann geöffnet oder verschlossen sein. Beispielsweise kann die Austragsöffnung erst kurz vor der Ausgabe der Flüssigkeit geöffnet werden, beispielsweise durch Abschneiden des verschließenden Endes der Auftragungshilfe. In der Regel ist die Austragsöffnung der Auftragungshilfe geöffnet. Auf diese Weise ist mit besonderem Vorteil eine Sterilisation des Innenraumes des äußeren Behältnisses, insbesondere der Außenseite des inneren Behältnisses, des erfindungsgemäßen Flüssigkeitsspenders möglich.

In einer bevorzugten Ausführungsform weist die Austragsöffnung einen lichten Querschnitt zwischen 0,2 und 3,0 mm², insbesondere zwischen 0,5 und 2,0 mm², insbesondere zwischen 0,5 und 1,0 mm², vorzugsweise von ca. 0,8 mm², auf. Austragsöffnungen mit derartigen lichten Querschnitten erlauben eine genaue und insbesondere zielgerichtete Auftragung der Flüssigkeit auf Wundareale. Dies ist insbesondere bei der Versorgung kleinerer Wunden von besonderem Vorteil. Der Öffnungsquerschnitt kann in gewünschter Weise geformt sein, beispielsweise kreisrund, oval oder flach.

Zwischen dem inneren Behältnis und der Austragsöffnung der Auftragungshilfe befindet sich eine poröse Barriere, welche für die aushärtbare Flüssigkeit durchlässig und für Bruchteile des inneren Behältnisses undurchlässig ist. Auf diese Weise kann für die Verabreichung der Flüssigkeit das innere Behältnis beispielsweise durch Druckausübung auf das äußere Behältnis zerstört werden, wobei Materialbestandteile des inneren Behältnisses durch die Barriere zurückgehalten werden.

In einer weitergehenden Ausführungsform besitzt die Barriere Poren mit einem Durchmesser zwischen 0,1 und 500 µm, vorzugsweise zwischen 10 und 200 µm. Bevorzugt ist die Barriere als poröse Membran ausgebildet, insbesondere in Form eines Filters. Ebenso kann es erfindungsgemäß vorgesehen sein, dass die Barriere als Schwamm oder Schaum ausgebildet ist.

Die Auftragungshilfe des erfindungsgemäßen Flüssigkeitsspenders weist vorzugsweise an ihrer Innenoberfläche Befestigungsmittel für die poröse Barriere auf. Bei den Befestigungsmitteln handelt es sich besonders bevorzugt um Ausbuchtungen in der Innenoberfläche der Auftragungshilfe. Zur Befestigung der Barriere kommen zweckmäßigerweise Klebe-, Schweiß- oder Cliptechniken in Frage.

Die poröse Barriere umhüllt das innere Behältnis vollständig. Die poröse Barriere ist als flüssigkeitsdurchlässige Umhüllung für das innere Behältnis ausgebildet. Bei der Umhüllung kann es sich beispielsweise um eine Schaumstoffummantelung handeln. Es ist ebenso möglich, dass die Umhüllung eine flüssigkeitsdurchlässige Folie ist, welche insbesondere Perforationen aufweisen kann. In einer bevorzugten Ausführungsform ist die Umhüllung als flüssigkeitsdurchlässiger Schlauch, beispielsweise als Wollschlauch, ausgebildet. Besonders bevorzugt ist die Umhüllung als flüssigkeitsdurchlässiger Schrumpfschlauch ausgebildet. Auf diese Weise kann das innere Behältnis nach seiner Abfüllung mit der aushärtbaren Flüssigkeit in besonders einfacher Weise in die Umhüllung überführt und darin eingeschlossen werden. Die Umhüllung kann auch aus Vliesmaterialien bestehen.

Als Materialien für die Umhüllung kommen insbesondere Kunststoffe, Gläser oder Keramiken in Frage. Zweckmäßigerweise ist die Umhüllung aus Polymeren, insbesondere aus Co- oder Terpolymeren, gebildet. Bei den Polymeren handelt es sich insbesondere um Polyolefine. Bevorzugt ist die Umhüllung des erfindungsgemäßen Flüssigkeitsspenders aus Polymeren auf der Basis von mindestens einem Monomer aus der Gruppe Polyethylen, Poylpropylen, Polystyrol, Polyurethan, Vinylidenchlorid, Vinylidenfluorid, Tetrafluorethylen und Hexafluoropropylen gebildet.

Das innere Behältnis besteht normalerweise aus gas- und dampfdichten Materialien und ist undurchlässig für Feuchtigkeit. In einer bevorzugten Ausführungsform ist das innere Behältnis aus einem zerbrechlichen Material, insbesondere aus Glas, gebildet. Die Wandstärke des inneren Behältnisses liegt zweckmäßigerweise zwischen 100 und 1000 µm, insbesondere zwischen 200 und 500 µm, vorzugsweise bei ca. 300 µm. Das innere Behältnis kann in unterschiedlichen Formen vorliegen. Beispielsweise kann das innere Behältnis zylinder- oder schlauchförmig ausgebildet sein und insbesondere ein abgeflachtes Ende aufweisen. Weiterhin kann das innere Behältnis spritzenförmig oder ampullenförmig ausgebildet sein.

In einer anderen Ausführungsform der Erfindung ist das innere Behältnis aus Metallen oder Verbundmaterialien gebildet, insbesondere in Form von Folien. Bei dem Metall handelt es sich vorzugsweise um Aluminium. Bevorzugt ist das Aluminium an der Innenoberfläche des inneren Behältnisses mit einem gegenüber der aushärtbaren Flüssigkeit inerten Material beschichtet. Somit wird mit besonderem Vorteil eine vorzeitige Aushärtung der Flüssigkeit vermieden.

Als Verbundmaterialien können grundsätzlich alle dem Fachmann geläufigen Materialien in Betracht kommen. Verbundmaterialien mit Metallen als Verbundkomponenten sind bevorzugt. Aluminium als Verbundkomponente ist wegen seiner Dampfdichtigkeit besonders geeignet. Die Verbundmaterialien können insbesondere einen schichtförmigen Aufbau besitzen. Die Verbundmaterialien können weiterhin beispielsweise Siegel- bzw. Klebeschichten enthalten. Mit Vorteil weisen die Verbundmaterialien sogenannte Laminate, insbesondere auf der Basis von Kunststoffen, auf. Bevorzugt sind Verbundmaterialien auf der Basis von Metallen, Papier, Kunststoffen und/oder Lacken. Die Verbundmaterialien können beispielsweise aus den folgenden Komponenten gebildet sein: Polyethylen, Polypropylen, Polyethylenterephthalat, Polyamid, Polyvinylchlorid, Lacke, Papier und/oder Aluminium. Besonders bevorzugt sind die im Folgenden aufgeführten Zusammensetzungen:
Polyethylen-Aluminium,-Polyethylenterephthalat, Polyvinylchlorid-Aluminium-Polyamid, Polypropylen-Aluminium-Lacke, Polyethylenterephthalat-Aluminium-Polypropylen und Lacke-Aluminium-Polyethylenterephthalat-Papier.

In einer bevorzugten Ausführungsform der Erfindung weist das innere Behältnis eine sogenannte Sollbruchstelle auf. Unter einer Sollbruchstelle im Sinne der vorliegenden Erfindung soll eine Schwachstelle des inneren Behältnisses verstanden werden, an welcher bei Einwirkung äußerer Kräfte auf den erfindungsgemäßen Flüssigkeitsspender in besonders leichter Weise Beschädigungen, insbesondere in Form von Brüchen, auftreten, wodurch die Freisetzung der aushärtbaren Flüssigkeit aus dem inneren Behältnis ermöglicht wird. Bei der Sollbruchstelle handelt es sich insbesondere um eine Verengung, beispielsweise wenn das innere Behältnis aus Glas besteht und insbesondere als Glasampulle vorliegt. Alternativ dazu handelt es sich bei der Sollbruchstelle vorzugsweise um eine schwache Schweißnaht. Dies ist besonders vorteilhaft, wenn das innere Behältnis aus einem Verbundmaterial oder aus Aluminium gebildet ist. Die Ausübung äußerer Kräfte auf den erfindungsgemäßen Flüssigkeitsspender bewirken in diesem Fall ein Aufplatzen der Schweißnaht, wodurch die aushärtbare Flüssigkeit aus dem inneren Behältnis freigesetzt wird.

Das äußere Behältnis des erfindungsgemäßen Flüssigkeitsspenders ist vorzugsweise aus einem flexiblen, insbesondere aus einem elastischen, Material gebildet. Als Materialien kommen zweckmäßigerweise Kunststoffe in Betracht. Das äußere Behältnis kann weiterhin in unterschiedlichen dreidimensionalen Ausbildungsformen vorliegen. So kann es sich bei dem äußeren Behältnis der Erfindung beispielsweise um eine Spritze, einen Zylinder, einen Schlauch oder eine Ampulle, insbesondere um eine Abdrehampulle, handeln.

In einer Ausführungsform ist die Auftragungshilfe von dem äußeren Behältnis getrennt ausgebildet. Die Auftragungshilfe ist vorzugsweise mit dem äußeren Behältnis konnektierbar ausgebildet. Für die Konnektierung der Auftragungshilfe und des äußeren Behältnisses kommen alle dem Fachmann geläufigen Konnektierungsmechanismen in Frage. So kann die Auftragungshilfe beispielsweise ein Innengewinde besitzen, welches komplementär zu einem entsprechenden Außengewinde des äußeren Behältnisses ist. Somit kann eine Konnektierung der Auftragungshilfe und des äußeren Behältnisses durch einfaches Verschrauben hergestellt werden.

In einer anderen Ausführungsform der Erfindung sind die Auftragungshilfe und das äußere Behältnis einstückig ausgebildet.

In einer bevorzugten Ausführungsform weist die Auftragungshilfe ein in den Innenraum des Flüssigkeitsspenders, insbesondere in den Innenraum des äußeren Behältnisses, gerichtetes nadelförmiges Element auf. Das nadelförmige Element ist vorzugsweise hohlzylindrisch ausgebildet, insbesondere nach Art einer Kanüle. Bei der Konnektierung der Auftragungshilfe und des äußeren Behältnisses wird das innere Behältnis, beispielsweise ein Aluminiumbeutel, unter Ausbildung vorzugsweise einer Austrittsöffnung von dem nadelförmigen Element der Auftragungshilfe perforiert. Besonders bevorzugt handelt es sich bei dem nadelförmigen Element um die Auftragungshilfe selbst, vorzugsweise in Form einer Kanüle. Somit dient das nadelförmige Element mit besonderem Vorteil einerseits zur Perforation des inneren Behältnisses und andererseits als Auftragungshilfe für die nach der Perforation des inneren Behältnisses austretende Flüssigkeit. Somit wird ein in seiner Handhabung besonders einfacher und komfortabler Flüssigkeitsspender bereitgestellt.

Es ist ebenso möglich, dass das innere Behältnis erst nach erfolgreicher Konnektierung der Auftragungshilfe und des äußeren Behältnisses von einem nadelförmigen Element perforiert wird. Beispielsweise kann nach der Konnektierung der Auftragungshilfe und des äußeren Behältnisses eine Nadel durch die hohle Auftragungshilfe hindurchgeführt werden, um das innere Behältnis zu perforieren.

Weiterhin können sich im Innenraum des äußeren Behältnisses scharfkantige Mittel befinden. Bei den scharfkantigen Mitteln kann es sich beispielsweise um Sägezähne handeln. Die scharfkantigen Mittel sind zweckmäßigerweise an der Innenoberfläche des äußeren Behältnisses befestigt. Durch Druckausübung auf das äußere Behältnis wird das innere Behältnis zumindest teilweise durch die scharfkantigen Mittel aufgerissen, so dass die innerhalb des inneren Behältnisses befindliche Flüssigkeit in den Innenraum des äußeren Behältnisses austreten kann.

In einer weiteren Ausführungsform liegt der Flüssigkeitsspender in einer Verpackung, beispielsweise in einer Tiefzieh- oder Peel-Verpackung, vor. Die Verpackung kann insbesondere aus einem gasdichten Material, beispielsweise aus Aluminium, gebildet sein. Erfindungsgemäß ist es ebenso möglich, dass die Verpackung aus gasdichten Verbundmaterialien, insbesondere mit Aluminium als Verbundkomponente, gebildet ist. Bezüglich weiterer Einzelheiten zu den Verbundmaterialien wird auf die bisherige Beschreibung verwiesen.

In einer bevorzugten Ausführungsform ist die Verpackung zumindest teilweise aus gasdurchlässigen und insbesondere keimdichten Materialien gebildet. In der Regel handelt es sich bei den Materialien um Faservliesmaterialien, insbesondere auf der Basis von Polyolefinen. Als bevorzugte Materialien kommen Polyethylen und/oder Polypropylen, insbesondere Hochdruckpolyethylen und/oder Hochdruckpolypropylen, in Betracht. Beispielsweise sind solche Materialien unter den Bezeichnungen Tyvek^{®} und Typar^{®} kommerziell erhältlich.

Der Flüssigkeitsspender liegt vorzugsweise sterilisiert vor, wobei bevorzugt die Außenseite des Flüssigkeitsspenders und insbesondere die Außenseite des inneren Behältnisses steril sind. Die Austragsöffnung der Auftragungshilfe ist zweckmäßigerweise zumindest während der Sterilisierung des Flüssigkeitsspenders geöffnet. Auf diese Weise ist auch eine Sterilisierung des Innenraumes des äußeren Behältnisses des Flüssigkeitsspenders möglich. Als Sterilisationsmethoden kommen insbesondere Plasma-, Ethylenoxid-, Wasserstoffperoxid- oder Dampfsterilisation in Frage. Zur Sterilisierung wird der Flüssigkeitsspender in der Regel in eine Verpackung überführt, welche insbesondere eine Sterilisationsöffnung aufweist. Bei der Sterilisationsöffnung kann es sich beispielsweise um eine Öffnung in der Verpackung handeln. Beispielsweise kann die Verpackung einseitig geöffnet sein. Bevorzugt handelt es sich bei der Sterilisationsöffnung um ein Wandungsteil der Verpackung, wobei die Sterilisationsöffnung vorzugsweise aus einem gasdurchlässigen und insbesondere keimdichten Material gebildet ist. Bezüglich des gasdichten und insbesondere keimdichten Materials wird auf die bisherige Beschreibung Bezug genommen. Die Sterilisationsöffnung ist zweckmäßigerweise gasdicht verschließbar. Als geeignete Verschlusstechniken, insbesondere nach beendeter Sterilisierung des Flüssigkeitsspenders, kommen beispielsweise Klebe- und Schweißtechniken in Frage. Erfindungsgemäß ist es weiterhin möglich, die Sterilisationsöffnung mit einem gasdichten Material, insbesondere in Form einer Folie, zu verschließen. Gegebenenfalls kann der Flüssigkeitsspender vor dem Verschluss der Sterilisationsöffnung mindestens einem Trocknungsschritt unterworfen werden. In einer besonders bevorzugten Ausführungsform ist der erfindungsgemäße Flüssigkeitsspender steril und frei von Restfeuchtigkeit.

Die aushärtbare Flüssigkeit des inneren Behältnisses ist vorzugsweise steril. Beispielsweise kann die Flüssigkeit steril filtriert sein. Bei der Flüssigkeit kann es sich insbesondere um eine Klebstoffzusammensetzung, insbesondere auf der Basis von Cyanoacrylat-Monomeren handeln. Die Cyanoacrylat-Monomere können im menschlichen und/oder tierischen Organismus resorbierbar oder nicht resorbierbar sein. Die Cyanoacrylat-Monomere können weiterhin als Mischung von verschiedenen Cyanaocrylat-Monomeren vorliegen. Bevorzugt weist die Klebstoffzusammensetzung Alkyl-Cyanoacrylat-Monomere und/oder Alkoxy-Alkyl-Cyanoacrylat-Monomere und/oder Alkylester-Cyanoacrylat-Monomere auf. Die Alkylketten der Cyanoacrylat-Monomere weisen insbesondere eine Kohlenstoffanzahl zwischen 1 bis 12, insbesondere zwischen 1 und 10, auf. Bei den Alkyl-Cyanoacrylat-Monomeren kann es sich beispielsweise um mindestens ein Monomer aus der Gruppe, umfassend Ethyl-, n-Butyl-, iso-Butyl- und n-Octyl-Cyanoacrylat handeln, wobei n-Butyl- und/oder n-Octylcyanoacrylat bevorzugt sind. Bei den Alkoxy-Alkyl-Cyanoacrylat-Monomeren handelt es sich insbesondere um Methoxypropyl- und/oder Ethoxyethyl-Cyanoacrylat, vorzugsweise um Methoxypropyl-Cyanoacrylat. Die Alkylester-Cyanoacrylat-Monomere sind bevorzugt aus der Gruppe, umfassend Butyllactoyl-, Butylglykoloyl-, IsopropyIglykoloyl-, Ethyllactoyl- und Ethylglykoloyl-Cyanoacrylat, ausgewählt.

Die aushärtbare Flüssigkeit kann zusätzliche Additive aufweisen. Bei den Additiven kann es sich insbesondere um Weichmacher handeln. Als Weichmacher kommen insbesondere alle dem Fachmann geläufigen Stoffe in Frage. Beispielsweise kommen als Weichmacher Fettsäurealkylester, insbesondere Isopropyl-Myristat, Ethyl-Myristat, Alkyl-Laureate, Alkyl-Palmitate, Diacetyladipat und/oder Butyl-Stearat, in Betracht. Bei den Weichmachern kann es sich weiterhin um Phthalsäureester, beispielsweise um Dioctylphthalat, Dibutylphthalat und/oder Butylbenzylphthalat, handeln. Als weitere Weichmacher kommen insbesondere Zitronensäureester, beispielsweise Tri-n-Butyl-Citrat, Acetyl-Trihexyl-ci-trat und/oder n-Butyl-Trihexylcitrat, in Frage.

Weiterhin kann die Flüssigkeit Verbindungen aufweisen, welche insbesondere die Viskosität der Flüssigkeit beeinflussen. In einer bevorzugten Ausführungsform weist die Flüssigkeit Polymere, insbesondere resorbierbare Polymere, auf. Die Polymere können insbesondere Co- und/oder Terpolymere sein, vorzugsweise auf der Basis von mindestens einem Monomer aus der Gruppe Lactid, Glykolid, Caprolacton, Trimethylencarbonat, p-Dioxanon und Hydroxybuttersäure. Besonders bevorzugt weist die Flüssigkeit ein Copolymer aus Lactid und Caprolacton auf. Weiterhin kann die Flüssigkeit ein Terpolymer auf der Basis von Trimethylencarbonat, Glykolid und Caprolacton aufweisen. Erfindungsgemäß kann es ebenso von Vorteil sein, dass die Flüssigkeit Poly-Dioxanon aufweist.

Erfindungsgemäß ist es insbesondere vorgesehen, dass die Flüssigkeit eine Mischung aus Cyanoacrylat-Monomeren und Polymeren, vorzugsweise resorbierbaren Polymeren, aufweist. Bezüglich weiterer Merkmale der Cyanoacrylat-Monomere sowie der Polymere wird auf die bisherige Beschreibung Bezug genommen.

Weiterhin kann die Flüssigkeit Stabilisatoren, insbesondere zur Verhinderung einer vorzeitigen Polymerisation von in der Flüssigkeit vorhandenen aushärtbaren Komponenten, aufweisen. Bevorzugt handelt es sich bei den Stabilisatoren um mindestens einen Stabilisator aus der Gruppe Schwefeldioxid, Phosphorsäure, Hydrochinon, tert.-ButylHydroxyanisol, Essigsäure und Schwefelsäure. Die einzelnen Stabilisatoren der Flüssigkeit können in unterschiedlichen Mengen vorhanden sein. Erfindungsgemäß liegen die Stabilisatoren bevorzugt in den folgenden Mengenbereichen vor: Schwefeldioxid: 20 bis 100 ppm, Phosphorsäure: 10 bis 500 ppm, Hydrochinon: 100 bis 3000 ppm, tert.-ButylHydroxyanisol: 100 bis 3000 ppm, Essigsäure: 10 bis 500 ppm und Schwefelsäure: 10 bis 500 ppm.

Die vorliegende Erfindung betrifft weiterhin die Verwendung des Flüssigkeitsspenders in der Medizin, insbesondere zur Behandlung von menschlichen und/oder tierischen Wunden. Bevorzugt handelt es sich bei den Wunden um innere Wunden. Bezüglich weiterer Merkmale und Einzelheiten zu dem Flüssigkeitsspender wird auf die bisherige Beschreibung Bezug genommen.

Weitere Merkmale der Erfindung ergeben sich aus den folgenden bevorzugten Ausführungsformen anhand von Figuren in Kombination mit den Unteransprüchen. Die Figuren werden hiermit durch ausdrückliche Bezugnahme zum Inhalt der Beschreibung gemacht.

In den Figuren ist folgendes schematisch (nicht maßstabsgetreu) gezeigt:
- Figur 1:: ein nicht erfindungsgemäßer Flüssigkeitsspender mit Polyurethanschaum,
- Figur 2:: Flüssigkeitsspender mit Schrumpfschlauch aus Polyethylen,
- Figur 3:: Flüssigkeitsspender mit Aluminiumbeutel,
- Figur 4:: ein nicht erfindungsgemäßer Flüssigkeitsspender in einer Verpackung mit einem einseitig integrierten Sterilisationsfenster.

### Figurenbeschreibung

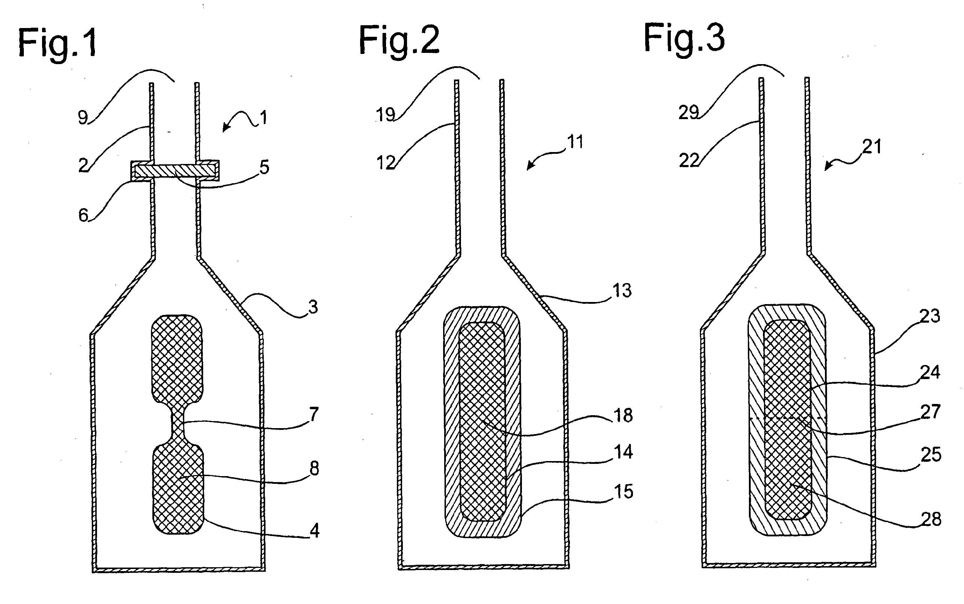

Figur 1 zeigt einen nicht erfindungsgemäßen Flüssigkeitsspender 1 aus einer als äußeres Behältnis ausgebildeten Kunststoffampulle 3 mit einer kanülenartigen Auftragungshilfe 2 und einer darin befindlichen und als separates inneres Behältnis ausgebildeten Glasampulle 4. Innerhalb der Auftragungshilfe 2 befindet sich ein als Barriere ausgebildeter offenporiger Polyethylenschaum 5. Die Auftragungshilfe 2 weist an ihrer Innenoberfläche Befestigungsmittel in Form von Ausbuchtungen 6 auf. Der Polyethylenschaum 5 ist in den Ausbuchtungen 6 arretiert, wodurch der Polyethylenschaum 5 beispielsweise gegen seitliches Verrutschen, insbesondere beim bestimmungsgemäßen Gebrauch des Flüssigkeitsspenders 1, gesichert ist. Die Glasampulle 4 weist eine Sollbruchstelle in Form einer Verengung 7 auf. Durch Ausüben von Druck auf die Kunststoffampulle 3 bricht die Glasampulle 4 im Bereich der Verengung 7 auf, wodurch eine darin als aushärtbare Flüssigkeit vorliegende flüssige Klebstoffzusammensetzung 8 auf der Basis von n-Butyl-Cyanoacrylat-Monomeren aus der Glasampulle 4 in den Innenraum der Kunststoffampulle 3 austritt. Durch den bestimmungsgemäßen Gebrauch des Flüssigkeitsspenders 1 fließt die freigesetzte Klebstoffzusammensetzung 8 durch den Polyethylenschaum 5 in die Auftragungshilfe 2 und schließlich durch die Austragsöffnung 9, die einen lichten Querschnitt von ca. 0,8 mm² aufweist, aus dem Flüssigkeitsspender 1.

Der Polyethylenschaum 5 übt eine Filterfunktion aus und hält die durch den Glasampullenbruch entstandenen Glassplitter zurück. Auf diese Weise ist eine unproblematische Auftragung der Klebstoffzusammensetzung 8 auf eine zu behandelnde Wunde möglich.

Figur 2 zeigt einen Flüssigkeitsspender 11 aus einer als äußeres Behältnis ausgebildeten Kunststoffampulle 13 mit einer kanülenartigen Auftragungshilfe 12 und einer darin befindlichen und als separates inneres Behältnis ausgebildeten Glasampulle 14. Die Glasampulle 14 wird von einem als poröse Barriere ausgebildeten Schrumpfschlauch 15 vollständig umhüllt und enthält eine aushärtbare Flüssigkeit in Form einer Klebstoffzusammensetzung 18 auf der Basis von n-Butyl- und n-Octyl-Cyanoacrylat-Monomeren. Bei Krafteinwirkung auf die Kunststoffampulle 13 wird die Glasampulle 14, deren Wandstärke ca. 300 µm beträgt, zerbrochen, wodurch die Klebstoffzusammensetzung 18 austritt. Die Klebstoffzusammensetzung 18 gelangt durch den porösen Schrumpfschlauch 15 in den Innenraum der Kunststoffampulle 13, während die Glassplitter durch den porösen Schrumpfschlauch 15 zurückgehalten werden. Durch den bestimmungsgemäßen Gebrauch des Flüssigkeitsspenders 11 kann die Klebstoffzusammensetzung 18 durch die Austragsöffnung 19 der Auftragungshilfe 12, welche einen lichten Querschnitt von ca. 0,8 mm² aufweist, auf den Wundbereich appliziert werden.

Figur 3 zeigt einen Flüssigkeitsspender 21 aus einer als äußeres Behältnis ausgebildeten Kunststoffampulle 23 mit einer kanülenartigen Auftragungshilfe 22 und einem als separates inneres Behältnis ausgebildeten Aluminiumbeutel 24 mit einer darin befindlichen aushärtbaren Flüssigkeit in Form einer Klebstoffzusammensetzung 28 auf der Basis von Alkoxy-Alkyl-Cyanoacrylat- und Alkylester-Cyanoacrylat-Monomeren. Der Aluminiumbeutel 24 weist eine Sollbruchstelle in Form einer schwachen Schweißnaht 27 auf. Durch äußere Krafteinwirkung auf die Kunststoffampulle 23 platzt der Aluminiumbeutel 24 im Bereich der schwachen Schweißnaht 27 auf, wodurch die Klebstoffzusammensetzung 28 in den Innenraum der Kunststoffampulle 23 gelangt. Durch den bestimmungsgemäßen Gebrauch des Flüssigkeitsspenders 21 kann die austretende Klebstoffzusammensetzung 28 durch die Austragsöffnung 29 mit einem lichten Querschnitt von ca. 0,8 mm² auf die zu versorgende Wunde aufgetragen werden.

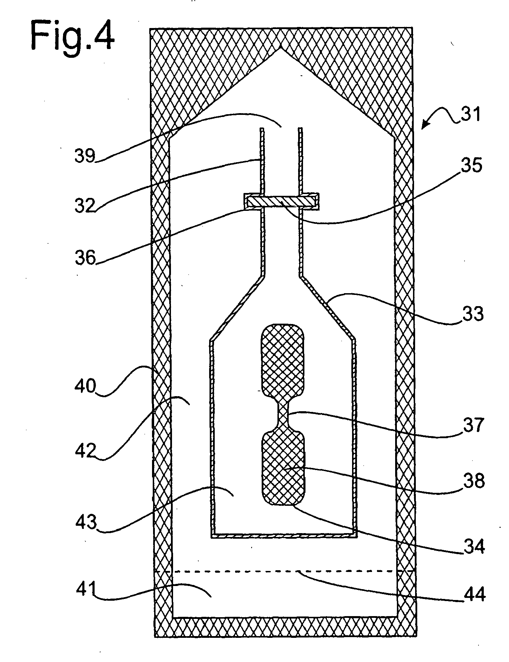

Figur 4 zeigt einen nicht erfindungsgemäßen Flüssigkeitsspender 31 aus einer als äußeres Behältnis ausgebildeten Kunststoffampulle 33 mit einer kanülenartigen Auftragungshilfe 32 und einer als separates inneres Behältnis ausgebildeten Glasampulle 34, welche eine Sollbruchstelle in Form einer Verengung 37 aufweist. Innerhalb der Glasampulle 34 befindet sich als aushärtbare Flüssigkeit eine Klebstoffzusammensetzung 38 auf der Basis von Alkyl- und Alkoxyalkyl-Cyanoacrylat-Monomeren. Innerhalb der Auftragungshilfe 32 befindet sich ein als Barriere ausgebildeter offenporiger Polyethylenschaum 35, welcher durch Befestigungsmittel in Form von Ausbuchtungen 36 an der Innenoberfläche der Auftragungshilfe 32 arretiert ist. Der Flüssigkeitsspender 31 befindet sich in einem als Verpackung ausgebildeten Aluminiumbeutel 40, welcher ein einseitig integriertes als Sterilisationsöffnung ausgebildetes Tyvek^{®}-Fenster 41 aufweist. Während des Sterilisationsvorganges gelangt das Sterilisationsgas durch das Tyvek^{®}-Fenster 41 in den Innenraum 42 des Aluminiumbeutels 40. Durch die offene Austragsöffnung 39 und den offenporigen Polyethylenschaum 35 gelangt das Sterilisationsgas weiter in den Innenraum 43 der Kunststoffampulle 33. Nach beendeter Sterilisierung wird der Aluminiumbeutel 40 entlang der Begrenzung 44 unter Entfernung des Tyvek^{®}-Fensters 41 durchgeschweißt, so dass der Flüssigkeitsspender 31 dampfdicht im Aluminiumbeutel 40 eingeschlossen ist, wobei der Innenraum 42 des Aluminiumbeutels 40 und insbesondere der Innenraum 43 der Kunststoffampulle 33 steril ist.

Durch die räumliche Trennung der Austragsöffnung 9, 19, 29 und 39 von der Barriere 5, 15 und 35 sowie von dem inneren Behältnis 4, 14, 24 und 34 kann die Austragsöffnung 9, 19, 29 und 39 beliebig gestaltet bzw. geformt werden, beispielsweise als kreisrunde oder als flache Düse. Dadurch ist eine Anpassung an die gewünschte Ausgabeart der aushärtbaren Flüssigkeit 8, 18, 28 und 38 möglich. Die Austragsöffnung 9, 19, 29 und 39 ist vorzugsweise geöffnet. Somit ist in besonders vorteilhafter Weise eine Sterilisierung des Flüssigkeitsspenders 1, 11, 21 und 31 nach Überführung in eine geeignete Verpackung 40 möglich. Vor dem gasdichten Verschließen der Verpackung 40 können Trocknungsschritte zwischengeschaltet werden. Auf diese Weise können Flüssigkeitsspender 1, 11, 21 und 31 bereitgestellt werden, die steril und insbesondere trocken, d.h. frei von Restfeuchtigkeit, sind. Das innere Behältnis 4, 14, 24 und 34 ist vorzugsweise in das äußere Behältnis 3, 13, 23 und 33 eingeschweißt.

## Patentansprüche

1. Flüssigkeitsspender (11; 21) in der Medizin in Form einer Handhabe mit einem äußeren Behältnis (13; 23), das eine hohle Auftragungshilfe (12; 22) aus einem nicht porösen Material, insbesondere nach Art einer Kanüle, aufweist, und einem inneren dampfdichten und geschlossenen Behältnis (14; 24) mit einer darin befindlichen aushärtbaren Flüssigkeit (18; 28), wobei die Auftragungshilfe (12; 22) eine Austragsöffnung (19; 29) mit einem lichten Querschnitt zwischen 0,1 und 5 mm² aufweist, **dadurch gekennzeichnet, dass** sich zwischen dem inneren Behältnis (14; 24) und der Austragsöffnung (19; 29) der Auftragungshilfe (12;22) eine poröse Barriere (15) befindet, welche für die aushärtbare Flüssigkeit durchlässig und für Bruchteile des inneren Behältnisses (14; 24) undurchlässig ist, wobei die poröse Barriere (15) das innere Behältnis (14; 24) vollständig umhüllt und als flüssigkeitsdurchlässige Umhüllung für das innere Behältnis (14; 24) ausgebildet ist.

2. Flüssigkeitsspender (11) nach Anspruch 1, **dadurch gekennzeichnet, dass** die Umhüllung (15) als poröse Membran oder poröser Schaum, insbesondere in Form eines Filters, ausgebildet ist.

3. Flüssigkeitsspender (11) nach Anspruch 1 oder 2, **dadurch gekennzeichnet, dass** die Umhüllung (15) für das innere Behältnis (14) als flüssigkeitsdurchlässiger Schlauch ausgebildet ist.

4. Flüssigkeitsspender (11) nach einem der vorhergehenden Ansprüche, **dadurch gekennzeichnet, dass** die Umhüllung (15) aus einem Polymer gebildet ist, insbesondere auf der Basis von mindestens einem Monomer aus der Gruppe Polyethylen, Polypropylen, Polystyrol, Polyurethan, Vinylidenchlorid, Vinylidenfluorid, Tetrafluorethylen und Hexafluoropropylen.

5. Flüssigkeitsspender (11) nach einem der vorhergehenden Ansprüche, **dadurch gekennzeichnet, dass** das innere Behältnis (14) aus einem zerbrechlichen Material, insbesondere aus Glas, gebildet ist.

6. Flüssigkeitsspender (21) nach Anspruch 1, **dadurch gekennzeichnet, dass** das innere Behältnis (24) aus Aluminium oder aus Verbundmaterialien, insbesondere mit Aluminium als Verbundkomponente, gebildet ist.

7. Flüssigkeitsspender (21) nach einem der vorhergehenden Ansprüche, **dadurch gekennzeichnet, dass** das innere Behältnis (24) eine Sollbruchstelle (27), insbesondere in Form einer Verengung oder einer auftrennbaren Schweißnaht, aufweist.

8. Flüssigkeitsspender (11; 21) nach einem der vorhergehenden Ansprüche, **dadurch gekennzeichnet, dass** die Auftragungshilfe (12; 22) getrennt von dem äußeren Behältnis (13; 23) und vorzugsweise mit dem äußeren Behältnis (13; 23) konnektierbar ausgebildet ist.

9. Flüssigkeitsspender (11; 21) nach einem der vorhergehenden Ansprüche 1 bis 7, **dadurch gekennzeichnet, dass** die Auftragungshilfe (12; 22) und das äußere Behältnis (13; 23) einstückig ausgebildet sind.

10. Flüssigkeitsspender (11; 21) nach einem der vorhergehenden Ansprüche, **dadurch gekennzeichnet, dass** die Auftragungshilfe (12; 22) ein in den Innenraum des Flüssigkeitsspenders (11; 21) gerichtetes nadelförmiges Element, insbesondere in Form einer Kanüle, aufweist.

11. Flüssigkeitsspender (11; 21) nach einem der vorhergehenden Ansprüche, **dadurch gekennzeichnet, dass** die aushärtbare Flüssigkeit (18; 28) eine Klebstoffzusammensetzung, insbesondere auf der Basis von Cyanoacrylat-Monomeren, ist.

## Claims

1. A liquid dispenser (11; 21) for medical purposes in the form of a handle having an outer container (13; 23), which comprises a hollow application aid (12; 22) made from a non-porous material, in particular in the manner of a cannula, and having an inner, vapour-tight and closed container (14; 24) with a curable liquid (18; 28) located therein, the application aid (12; 22) having a delivery opening (19; 29) with a clear cross section of between 0.1 and 5 mm²,
**characterised in that**
located between the inner container (14; 24) and the delivery opening (19; 29) of the application aid (12; 22), there is a porous barrier (15) which is pervious for the curable liquid and impervious for fragments of the inner container (14; 24), wherein the porous barrier (15) completely encloses the inner container (14; 24) and is a liquid-pervious envelope for the inner container (14; 24).

2. The liquid dispenser (11) according to claim 1, **characterised in that** the envelope (15) is designed as a porous membrane or porous foam, in particular in the form of a filter.

3. The liquid dispenser (11) according to claim 1 or 2, **characterised in that** the envelope (15) for the inner container (14) is designed as a liquid-pervious flexible tube.

4. The liquid dispenser (11) according to any one of the preceding claims, **characterised in that** the envelope (15) is formed from a polymer, based in particular on at least one monomer from the group consisting of polyethylene, polypropylene, polystyrene, polyurethane, vinylidene chloride, vinylidene fluoride, tetrafluoroethylene, and hexafluoropropylene.

5. The liquid dispenser (11) according to any one of the preceding claims, **characterised in that** the inner container (14) is formed from a frangible material, in particular from glass.

6. The liquid dispenser (21) according to claim 1, **characterised in that** the inner container (24) is formed from aluminum or from composite materials, in particular with aluminum as a component of the composite.

7. The liquid dispenser (21) according to any one of the preceding claims, **characterised in that** the inner container (24) has a predetermined breakage point (27), in particular in the form of a constriction or a separable weld seam.

8. The liquid dispenser (11; 21) according to any one of the preceding claims, **characterised in that** the application aid (12; 22) is designed in such a way that it is separate from the outer container (13; 23) and preferably connectable with the outer container (13; 23).

9. The liquid dispenser (11; 21) according to any one of the preceding claims 1 to 7, **characterised in that** the application aid (12; 22) and the outer container (13; 23) are of one-piece design.

10. The liquid dispenser (11; 21) according to any one of the preceding claims, **characterised in that** the application aid (12; 22) has a needle-shaped element, in particular in the form of a cannula, which points into the interior of the liquid dispenser (11; 21).

11. The liquid dispenser (11; 21) according to any one of the preceding claims, **characterised in that** the curable liquid (18; 28) is an adhesive composition, based in particular on cyanoacrylate monomers.

## Revendications

1. Distributeur de fluide (11 ; 21) utilisé en médecine sous la forme d'un manche comprenant un récipient extérieur (13 ; 23), qui présente un auxiliaire d'application creux (12 ; 22) constitué d'un matériau non poreux, en particulier de type canule, et un récipient intérieur (14 ; 24) étanche à la vapeur et fermé contenant un fluide durcissable (18 ; 28), l'auxiliaire d'application (12 ; 22) présentant une ouverture de sortie (19 ; 29) avec une section transversale intérieure comprise entre 0,1 et 5 mm², **caractérisé en ce qu'**entre le récipient intérieur (14 ; 24) et l'ouverture de sortie (19 ; 29) de l'auxiliaire d'application (12 ; 22) se trouve une barrière poreuse (15) qui est perméable au fluide durcissable et imperméable à des fractions du récipient intérieur (14 ; 24), la barrière poreuse (15) enveloppant complètement le récipient intérieur (14 ; 24) et étant réalisée en tant qu'enveloppe perméable aux fluides pour le récipient intérieur (14 ; 24).

2. Distributeur de fluide (11) selon la revendication 1, **caractérisé en ce que** l'enveloppe (15) est réalisée sous forme de membrane poreuse ou de mousse poreuse, en particulier sous forme de filtre.

3. Distributeur de fluide (11) selon la revendication 1 ou 2, **caractérisé en ce que** l'enveloppe (15) pour le récipient intérieur (14) est réalisée sous forme de tuyau souple perméable aux fluides.

4. Distributeur de fluide (11) selon l'une quelconque des revendications précédentes, **caractérisé en ce que** l'enveloppe (15) est formée d'un polymère, en particulier à base d'au moins un monomère du groupe comprenant du polyéthylène, du polypropylène, du polystyrène, du polyuréthane, du chlorure de vinylidène, du fluorure de vinylidène, du tétrafluoréthylène, et de l'hexafluoropropylène.

5. Distributeur de fluide (11) selon l'une quelconque des revendications précédentes, **caractérisé en ce que** le récipient intérieur (14) est formé d'un matériau cassant, en particulier de verre.

6. Distributeur de fluide (21) selon la revendication 1, **caractérisé en ce que** le récipient intérieur (24) est formé d'aluminium ou de matériaux composites, en particulier d'aluminium en tant que composant composite.

7. Distributeur de fluide (21) selon l'une quelconque des revendications précédentes, **caractérisé en ce que** le récipient intérieur (24) présente un point destiné à la rupture (27), en particulier sous forme d'un rétrécissement ou d'un joint de soudure séparable.

8. Distributeur de fluide (11 ; 21) selon l'une quelconque des revendications précédentes, **caractérisé en ce que** l'auxiliaire d'application (12 ; 22) est réalisé de manière séparée du récipient extérieur (13 ; 23) et de préférence de manière à pouvoir être connecté au récipient extérieur (13 ; 23).

9. Distributeur de fluide (11 ; 21) selon l'une quelconque des revendications précédentes 1 à 7, **caractérisé en ce que** l'auxiliaire d'application (12 ; 22) et le récipient extérieur (13 ; 23) sont réalisés d'une seule pièce.

10. Distributeur de fluide (11 ; 21) selon l'une quelconque des revendications précédentes, **caractérisé en ce que** l'auxiliaire d'application (12 ; 22) présente un élément en forme d'aiguille orienté dans l'espace interne du distributeur de fluide (11 ; 21), en particulier sous la forme d'une canule.

11. Distributeur de fluide (11 ; 21) selon l'une quelconque des revendications précédentes, **caractérisé en ce que** le fluide durcissable (18 ; 28) est une composition d'adhésif, en particulier à base de monomères de cyanoacrylate.
